# DEMANDE DE BREVET EUROPEEN

(11) **EP 4 375 638 A1**
(43) Date de publication de la demande: **29.05.2024**
(21) Numéro de dépôt: 23212219.2
(22) Date de dépôt: 27.11.2023
(51) Int. Cl.: G01N 1/40, G01N 21/76, G01N 27/32, G01N 33/02

(54) **PROCÉDÉ DE DÉTECTION DU SCATOL DANS UN TISSU ADIPEUX DE PORC MÂLE**

(30) Priorité: 28.11.2022 FR 2212395
(71) Demandeur: Commissariat à l'énergie atomique et aux énergies alternatives, 75015 Paris (FR)
(72) Inventeur: STEWART, Samuel, 91191 Gif-sur-Yvette Cedex (FR); HAMEL, Matthieu, 91191 Gif-sur-Yvette Cedex (FR); SCORSONE, Emmanuel, 91191 Gif-sur-Yvette Cedex (FR)
(74) Mandataire: Brevalex

(57) **Abrégé**

L'invention se rapporte à un procédé pour détecter la présence de scatol dans un tissu adipeux d'un porc mâle, qui comprend au moins les étapes consistant à :
a) préparer un extrait organique à partir d'un échantillon du tissu adipeux ;
b) soumettre l'extrait organique préparé à l'étape a) à une réaction d'électrochimioluminescence ; et
c) mesurer l'intensité de la luminescence pendant l'étape b) et, si l'intensité de luminescence mesurée dépasse une valeur seuil prédéterminée, déduire la présence de scatol dans l'échantillon de tissu adipeux ;
et qui est caractérisé en ce que l'étape a) comprend les sous-étapes consistant à :
i) prélever l'échantillon de tissu adipeux à l'aide d'un écouvillon de prélèvement ;
ii) dissoudre l'échantillon de tissu adipeux dans un milieu comprenant un solvant organique aprotique ;
iii) ajouter un sel de fond anhydre au solvant organique aprotique, cet ajout pouvant être réalisé avant ou après la sous-étape ii).

Applications : identification, sur les chaînes d'abattage, des porcs mâles entiers dont la viande est porteuse de l'odeur de verrat ; outil de recherche, notamment pour étudier les facteurs influençant la production de scatol dans les tissus adipeux des porcs mâles entiers en vue de développer des méthodes permettant de prévenir ou de réduire l'odeur de verrat.

## Description

### DOMAINE TECHNIQUE

L'invention relève du domaine de l'agroalimentaire et, en particulier, du domaine de la production et de la distribution de la viande de porc.

Plus spécifiquement, l'invention se rapporte à un procédé permettant de détecter très rapidement la présence de scatol, ou 3-méthylindole (3-MIH), dans un tissu adipeux de porc mâle et, si celui-ci est présent, d'en déterminer la teneur et ce, avec une très grande sensibilité et une très grande spécificité vis-à-vis des autres composés susceptibles d'être présents dans ce tissu adipeux.

Le scatol étant, avec l'androsténone, responsable d'une odeur forte et désagréable, dite odeur de verrat, qui est dégagée pendant la cuisson de la viande de porcs mâles entiers, l'invention est, en premier lieu, susceptible d'être mise en oeuvre pour trier dans les abattoirs les carcasses de porcs mâles entiers en vue d'isoler celles dont la viande est porteuse d'odeur de verrat et de les orienter vers un circuit de transformation adapté.

Elle est également susceptible d'être mise en oeuvre comme outil de recherche, notamment pour étudier les facteurs influençant la production de scatol dans les tissus adipeux des porcs mâles entiers en vue de développer des méthodes permettant de prévenir ou de réduire l'odeur de verrat, par exemple par sélection génétique ou par modification des conditions d'élevage (alimentation, conditions de stabulation, composition des groupes d'animaux en termes d'âge, de sexe, etc.).

### ÉTAT DE LA TECHNIQUE ANTÉRIEURE

L'odeur de verrat découle principalement d'une accumulation de scatol et de l'androsténone et, dans une moindre mesure, de l'indole dans les tissus adipeux - ou tissus gras - des porcs mâles entiers, c'est-à-dire non castrés.

Cette odeur, qui se dégage pendant la cuisson de la viande de porc, est généralement considérée comme nauséabonde par les consommateurs.

Historiquement, pour prévenir l'odeur de verrat, les porcelets mâles étaient castrés avant qu'ils n'atteignent une maturité sexuelle.

Toutefois depuis une quinzaine d'années, des raisons éthiques, de bien-être animal mais également économiques amènent de plus en plus d'éleveurs à ne plus castrer les porcelets mâles et ce, d'autant plus que seuls 5 % à 10 % des adultes développent l'odeur de verrat.

On connaît un certain nombre de procédés permettant d'identifier, sur les chaînes d'abattage, les carcasses de porcs mâles dont la viande est porteuse de l'odeur de verrat.

En particulier, il a été décrit dans la demande internationale PCT WO-A-2021/009438, ci-après référence [1], un procédé permettant de détecter et de doser le scatol présent dans un échantillon de tissu adipeux de porc avec une très grande sensibilité - puisque ce procédé présente une limite de détection de l'ordre de 20 nmol/L d'échantillon - et une très grande spécificité vis-à-vis des autres composés indoliques susceptibles d'être également présents dans ce tissu adipeux.

Ce procédé qui a, en outre, pour avantage de pouvoir être mis en oeuvre en abattoir, consiste à préparer un extrait organique de l'échantillon de tissu adipeux et à soumettre cet extrait à une réaction d'électrochimioluminescence (ECL).

La préparation de l'extrait organique de l'échantillon de tissu adipeux, qui est proposée dans la référence [1], comprend une succession d'étapes, à savoir : une séparation du gras présent dans l'échantillon de tissu adipeux des éléments non graisseux également présents dans cet échantillon, une déshydratation du gras présent dans l'échantillon de tissu adipeux, une dissolution du gras déshydraté dans un solvant organique aprotique, un chauffage de la solution organique résultant de cette dissolution pour permettre au scatol d'être extrait, un dégraissage de la solution organique et, enfin, un ajout d'un sel de fond anhydre à cette solution organique.

Il s'avère que, compte-tenu de la cadence d'abattage à laquelle sont soumis les abattoirs, le laps de temps qui sépare l'abattage des porcs de l'expédition des carcasses vers les ateliers de découpe et les filières de transformation est très court.

Or, l'identification des carcasses (qui peuvent être au nombre de plusieurs centaines par jour) dont la viande est porteuse de l'odeur de verrat doit être réalisée pendant ce laps de temps très court.

Il en résulte que le temps nécessaire pour détecter la présence de scatol dans ces carcasses et, s'il est présent, pour le doser doit être optimisé.

### EXPOSÉ DE L'INVENTION

L'invention porte sur un perfectionnement du procédé décrit dans la référence [1], qui permet justement de réduire au mieux le temps nécessaire pour détecter la présence de scatol dans un tissu adipeux de porc mâle et, le cas échéant, doser ce scatol par une simplification de la préparation des extraits organiques destinés à être soumis à la réaction d'électrochimioluminescence et ce, sans que les performances de ce procédé, en termes de sensibilité et de spécificité, en soient affectées.

L'invention se rapporte donc à un procédé pour détecter la présence de scatol dans un tissu adipeux d'un porc mâle, qui comprend au moins les étapes consistant à :
a) préparer un extrait organique à partir d'un échantillon du tissu adipeux ;
b) soumettre l'extrait organique préparé à l'étape a) à une réaction d'électrochimioluminescence ; et
c) mesurer l'intensité de la luminescence pendant l'étape b) et, si l'intensité de luminescence mesurée dépasse une valeur seuil prédéterminée, déduire la présence de scatol dans l'échantillon de tissu adipeux ;
et qui est caractérisé en ce que l'étape a) comprend les sous-étapes consistant à :
i) prélever l'échantillon de tissu adipeux à l'aide d'un écouvillon de prélèvement ;
ii) dissoudre l'échantillon de tissu adipeux dans un milieu comprenant un solvant organique aprotique ;
iii) ajouter un sel de fond anhydre au solvant organique aprotique, cet ajout pouvant être réalisé avant ou après la sous-étape ii) ;
moyennant quoi on obtient l'extrait organique.

Ainsi, selon l'invention, l'échantillon de tissu adipeux est prélevé au moyen d'un écouvillon de prélèvement puis dissous ou mis en solution (les deux termes étant considérés comme synonymes) dans un milieu comprenant un solvant organique aprotique et, éventuellement, un sel de fond anhydre. Si le sel de fond anhydre n'est pas déjà présent dans le milieu au moment de la dissolution de l'échantillon, alors il peut être ajouté après cette dissolution.

L'expression « écouvillon de prélèvement » désigne tout écouvillon du type de ceux classiquement utilisés pour réaliser des prélèvements d'échantillons biologiques ou microbiologiques tel qu'un coton-tige de laboratoire et, plus généralement, tout bâtonnet en plastique, en bois, en aluminium ou autre, garni à l'une de ses extrémités d'un embout en une matière absorbante, cette matière pouvant être naturelle (coton ou ouate par exemple) ou artificielle (mousse de viscose ou de polyester par exemple).

Conformément à l'invention, le prélèvement est, de préférence, réalisé en frottant le tissu adipeux avec l'écouvillon pendant un temps suffisant pour saturer l'écouvillon en tissu adipeux. Ce temps dépendra évidemment de la taille de l'embout de l'écouvillon utilisé pour le prélèvement mais, généralement, 20 secondes d'un frottement continu seront suffisantes pour obtenir une saturation de cet embout.

Ensuite, une fois que l'échantillon de tissu adipeux est prélevé au moyen de l'écouvillon, il est mis en solution afin de pouvoir être analysé par la réaction d'ECL.

Dans ce qui précède et ce qui suit, le terme « aprotique », lorsqu'il est appliqué à un solvant organique, est pris dans son acception habituelle, à savoir qu'il désigne un solvant organique dont la molécule est exempte d'atome d'hydrogène acide, c'est-à-dire lié à un hétéroatome comme un atome d'azote, d'oxygène ou de soufre.

Conformément à l'invention, le solvant organique aprotique utilisé à la sous-étape ii) est avantageusement un solvant aprotique polaire, c'est-à-dire présentant un moment dipolaire non nul, comme l'acétonitrile, le diméthylsulfoxyde (ou DMSO), le carbonate de propylène ou la γ-butyrolactone, préférence étant donnée à l'acétonitrile.

Le sel de fond anhydre, qui est ajouté au solvant organique aprotique, peut être choisi parmi de très nombreux sels étant entendu qu'il doit être, d'une part, soluble dans le solvant organique aprotique et, d'autre part, chimiquement et électrochimiquement inerte de sorte à ne pas perturber la réaction d'ECL ni induire une réaction indésirable avec le scatol.

Comme bien connu des électrochimistes, ce sel peut notamment être un tétrafluoroborate, un hexafluorophosphate ou un perchlorate de tétraalkylammonium dont le groupe alkyle comprend de 1 à 6 atomes de carbone, tel que le tétrafluoroborate de tétrabutylammonium ou l'hexafluorophosphate de tétrabutylammonium (ou TBAHFP), ce type de sel présentant, en effet, une stabilité remarquable en milieu organique, préférence étant donnée au TBAHFP.

Par ailleurs, le sel de fond anhydre est ajouté au solvant organique aprotique en une quantité telle que sa concentration dans ce solvant soit typiquement comprise entre 0,01 mol/L et 1 mol/L, de préférence entre 0,05 mol/L et 0,5 mol/L, et, mieux encore, égale à 0,1 mol/L.

Conformément à l'invention, on préfère que l'extrait organique préparé à l'étape a) comprenne au plus 0,1 % volumique d'eau.

En effet, au-dessus de ce seuil, si du scatol est présent dans l'extrait organique - le porc mâle étant contaminé en scatol - l'intensité du signal de luminescence mesurée par la réaction d'ECL risque d'être affaiblie du fait de l'interaction entre l'eau et les ions superoxydes, interférant alors dans la formation de la base conjuguée du scatol selon la réaction suivante :

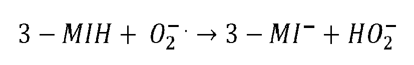

Aussi, sachant qu'un tissu adipeux de porc contient de 10 % à 20 % massiques d'eau, il peut être nécessaire, en fonction de la masse de l'échantillon de tissu adipeux prélevée, d'ajouter de plus au solvant organique aprotique, soit avant la sous-étape ii) soit après la sous-étape ii), un agent déshydratant ou agent de séchage (les deux termes étant considérés comme synonymes) tel qu'un sel hygroscopique non soluble dans le solvant organique aprotique du type sulfate de sodium anhydre, sulfate de potassium anhydre ou sulfate de magnésium anhydre, ou un tamis moléculaire (par exemple, de 3 ou 4 angströms) pour éliminer tout ou partie de l'eau susceptible d'être libérée lors de la dissolution de l'échantillon de tissu adipeux.

Si tel est le cas, cet agent déshydratant peut ensuite être éliminé du milieu de dissolution, par exemple par décantation, filtration ou par centrifugation, avant de procéder à l'étape b).

Conformément à l'invention, on préfère faciliter la dissolution de l'échantillon de tissu adipeux au moyen d'un agent abrasif que l'on ajoute au solvant organique aprotique avant de procéder à la sous-étape ii).

Dans ce qui précède et ce qui suit, l'expression « agent abrasif » s'entend de toute matière insoluble dans le solvant organique aprotique et se présentant sous une forme très finement divisée qui, lorsqu'elle est présente dans le milieu comprenant le solvant organique aprotique, est capable d'arracher par simple frottement le tissu adipeux de l'écouvillon. Ainsi, il peut notamment s'agir d'une poudre constituée d'un sel insoluble dans le solvant organique aprotique tel qu'un sel d'un métal alcalin ou alcalino-terreux du type sulfate de sodium, sulfate de potassium, sulfate de magnésium ou d'une poudre qui n'est pas constituée d'un sel telle qu'une poudre de silice, une poudre d'un oxyde métallique (alumine par exemple), une poudre de pierre ponce, une poudre de terre de diatomée, etc..

Parmi ces agents abrasifs, préférence est donnée à un agent abrasif qui peut faire aussi office d'agent déshydratant, ce qui est notamment le cas d'un sel hygroscopique comme le sulfate de sodium anhydre, le sulfate de potassium anhydre ou le sulfate de magnésium anhydre.

Typiquement, l'agent abrasif est ajouté au solvant organique à hauteur de 0,5 g à 1 g d'agent abrasif pour 10 mL de solvant.

Si un agent abrasif est ajouté au solvant organique aprotique, alors la mise en solution de l'échantillon de tissu adipeux est, de préférence, réalisée en introduisant l'écouvillon dans un récipient (typiquement de forme cylindrique ou conique du type tube à essai, pilulier ou tube à centrifuger) dans lequel se trouve le milieu comprenant le solvant organique aprotique, l'agent abrasif et, éventuellement, le sel de fond anhydre, en immergeant l'écouvillon dans ce milieu et en le frottant, par exemple par rotation, contre la paroi du récipient pendant au moins 20 secondes.

Une mise sous agitation du récipient, par exemple au moyen d'un agitateur de laboratoire de type vortex, peut avantageusement compléter ce protocole.

Dans tous les cas, l'agent abrasif peut ensuite être éliminé du milieu de dissolution, par exemple par décantation, filtration ou par centrifugation, avant de procéder à l'étape b).

Comme connu en soi, la réaction d'ECL est, de préférence, réalisée dans une cellule électrochimique, le terme « cellule électrochimique » désignant ici l'ensemble formé par un contenant de type cuvette ou analogue, dans lequel est placé l'extrait organique pour la réaction d'ECL, et au moins deux électrodes, à savoir une électrode de travail et une contre-électrode.

Conformément à l'invention, on préfère ;
- que la réaction d'ECL soit initiée par l'application d'un potentiel cathodique à une électrode de travail, qui est plongée dans l'extrait organique, de sorte à induire la formation d'ions superoxydes par réduction du dioxygène dissous dans cet extrait, puis la formation de la base conjuguée du scatol et de radicaux hydroperoxydes ; et
- que l'application d'un potentiel cathodique soit suivie de l'application d'un potentiel anodique de sorte à oxyder la base conjuguée du scatol qui, une fois oxydée, va réagir avec les radicaux hydroperoxydes pour conduire à la formation de N-5(2-acétyl-phényl)formamide à l'état excité lequel par désexcitation (ou, autrement dit, par retour à son état fondamental), émet une luminescence mesurable.

Pour des détails sur ce processus, le lecteur est invité à se référer à la référence [1] ainsi qu'à l'article publié par T. Okajima et T. Ohsaka dans Journal of Electroanalytical Chemistry 2002, 523, 34-39, ci-après référence [2].

L'application à l'électrode de travail d'un potentiel cathodique puis d'un potentiel anodique peut être réalisée selon différents protocoles électrochimiques et, notamment, par :
- un balayage en potentiel, auquel cas on applique à l'électrode de travail un balayage vers les potentiels négatifs puis un balayage vers les potentiels positifs ;
- un saut de potentiel, auquel cas on applique à l'électrode de travail un potentiel négatif constant, pendant une durée suffisante pour saturer la surface de cette électrode en ions superoxydes, puis un potentiel positif constant, également pendant une durée suffisante pour saturer la surface de l'électrode de travail en scatol oxydé ; ou
- par une série d'impulsions de potentiel alternativement cathodique et anodique.

Toutefois, dans le cadre de l'invention, on préfère que l'application à l'électrode de travail d'un potentiel cathodique puis d'un potentiel anodique soit réalisée par saut de potentiel car il s'agit du protocole électrochimique qui permet de réaliser la réaction d'ECL le plus rapidement.

À titre d'exemple, pour une cellule électrochimique munie d'une électrode de travail et d'une contre-électrode en dimant dopé au bore ainsi que d'une électrode de pseudo-référence en platine, d'excellents résultats ont été obtenus en appliquant à l'électrode de travail un potentiel négatif constant inférieur à -1,5 V, par exemple de -1,8 V, pendant de 10 secondes à 60 secondes, puis un potentiel positif constant supérieur à +0,5 V, par exemple de +0,8 V, pendant de 1 à 15 secondes.

Le choix du contenant de la cellule électrochimique n'est pas critique en soi.

Toutefois, il est souhaitable que ce contenant soit en un matériau résistant aux solvants organiques et aux milieux salins.

Par ailleurs, il convient que ce contenant soit en un matériau optiquement transparent dans la gamme des longueurs d'onde d'émission de la luminescence ou qu'il présente au moins une paroi en un matériau présentant une telle transparence si la détection des photons émis est réalisée par un détecteur optique situé en vis-à-vis de l'une de ses parois.

Le choix des électrodes de la cellule électrolytique n'est pas critique non plus.

Si la réaction d'ECL est basée sur la formation d'ions superoxydes, alors l'électrode de travail peut être constituée de tout matériau d'électrode permettant la formation de tels ions comme du carbone (graphite, carbone vitreux, diamant dopé, par exemple au bore ou à l'azote, etc.), un métal noble (or, platine, palladium, iridium, etc.) ou un alliage de métaux nobles.

La contre-électrode peut être constituée d'un matériau d'électrode différent ou du même matériau d'électrode que celui qui constitue l'électrode de travail.

Dans le cadre de l'invention, on préfère que l'électrode de travail et la contre-électrode soient en diamant dopé, notamment au bore, en raison de ce que ce matériau est hautement conducteur, présente une très grande stabilité, une résilience naturelle à l'encrassement due à la forte densité atomique du diamant. Cette résilience à l'encrassement est particulièrement intéressante compte-tenu que l'extrait organique peut comprendre un certain nombre de composés issus du tissu adipeux de porc, dont des acides gras, qui peuvent encrasser rapidement la surface des électrodes. De plus, en cas d'encrassement, ce type d'électrode peut être aisément décrassé par voie électrochimique, par exemple par le procédé décrit dans le brevet US 9,121,107 B2, ci-après référence [3]. Enfin, ce type d'électrode présente une grande fenêtre de potentiel qui permet d'appliquer des potentiels élevés sans venir électrolyser le solvant présent dans l'extrait organique.

Si une électrode de référence est utilisée, alors celle-ci peut notamment être une électrode au calomel saturé (ECS) ou une électrode au chlorure d'argent (Ag/AgCl), éventuellement à double jonction, telles que traditionnellement utilisées en électrochimie. Toutefois, dans le cadre de l'invention, on préfère utiliser une électrode de pseudo-référence en un métal noble tel que le platine parce que ce type d'électrode peut également être aisément décrassé, par exemple par passage à la flamme.

Avantageusement, la cellule électrochimique (i.e. contenant et électrodes) est réalisée dans des matériaux à bas coûts (contenant en plastique, électrodes à pâte de carbone, etc.) de sorte à être à usage unique, ce qui permet, d'une part, de s'affranchir des problèmes d'encrassement des électrodes et, d'autre part, d'assurer une traçabilité des échantillons de tissus adipeux analysés, par exemple en référençant chaque cellule électrochimique par rapport à une carcasse de porc.

Quant au détecteur optique, il peut s'agir d'un photomultiplicateur couplé à une photocathode en bialkali, super-bialkali ou ultra-bialkali, d'une photodiode à avalanche, d'un photomultiplicateur à photocathode en silicium, d'un spectromètre et, notamment, un spectrofluorimètre, d'un détecteur à capteur CCD (de « Charged Coupled Device »), d'un détecteur à capteur CMOS (de « Complementarity Metal-Oxide-Semiconductor »), etc.

Conformément à l'invention, la valeur seuil utilisée à l'étape c) est, de préférence, au moins égale à 150 % de la valeur moyenne de l'intensité de la luminescence correspondant au bruit de fond du détecteur optique.

Comme précédemment indiqué, le procédé de l'invention permet aussi, si du scatol est présent dans l'extrait organique préparé à l'étape a), d'en déterminer la concentration.

Ainsi, si la présence de scatol a été déduite à l'étape c), le procédé comprend avantageusement de plus une quantification du scatol présent dans l'extrait organique par comparaison de l'intensité maximale de luminescence mesurée au cours de l'étape c) avec une courbe d'étalonnage, cette quantification étant réalisée au cours de l'étape c) ou postérieurement à l'étape c).

Le seuil de rejet d'une viande de porc par le consommateur est fixé à environ 0,2 µg de scatol par gramme de tissu adipeux, ce qui correspond, dans l'hypothèse d'une extraction complète du scatol présent dans 1g de tissu adipeux dans 1 mL d'un solvant organique à une concentration en scatol de 1,53 µmol/L.

Aussi, l'invention a-t-elle également pour objet un procédé de tri de carcasse de porcs mâles entiers, qui est caractérisé en qu'il comprend la mise en oeuvre d'un procédé de détection du scatol tel que précédemment défini.

D'autres caractéristiques et avantages de l'invention ressortiront du complément de description qui suit, qui se rapporte à des expérimentations ayant permis de valider l'invention et qui est donné en référence aux figures annexées.

Il va de soi que ce complément de description n'est donné qu'à titre d'illustration de l'objet de l'invention et ne doit en aucun cas être interprété comme une limitation de cet objet.

### BRÈVE DESCRIPTION DES FIGURES

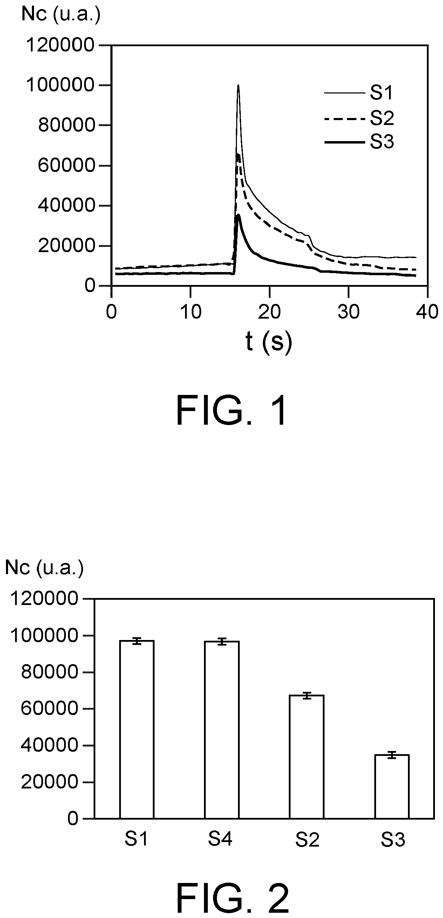

La figure 1 illustre les signaux de luminescence obtenus en soumettant, à une réaction d'ECL par saut de potentiel, 3 solutions synthétiques, respectivement, S1, S2 et S3, comprenant toutes 100 nmol/L de scatol dans un milieu comprenant de l'acétonitrile et 0,1 mol/L de TBAHFP mais différant les unes des autres par leur pourcentage volumique en eau (respectivement 0 %, 1 % et 2 %) ; sur cette figure, l'axe des ordonnées correspond au nombre de coups émis, noté Ne et exprimé en unités arbitraires (u.a.) tandis que l'axe des abscisses correspond à la durée, notée t et exprimée en secondes, de la réaction d'ECL.
La figure 2 illustre, sous la forme d'un diagramme en bâtons, l'intensité maximale des signaux de luminescence obtenus en soumettant, à une réaction d'ECL par saut de potentiel, les solutions S1, S2 et S3 ainsi qu'une quatrième solution synthétique, S4, comprenant comme les précédentes 100 nmol/L de scatol dans un milieu comprenant de l'acétonitrile et 0,1 mol/L de TBAHFP mais différant de celles-ci en ce qu'elle comprend 0,1 % volumique d'eau; sur cette figure, l'axe des ordonnées correspond au nombre maximal de coups émis, noté Ne et exprimé en unités arbitraires (u.a.).

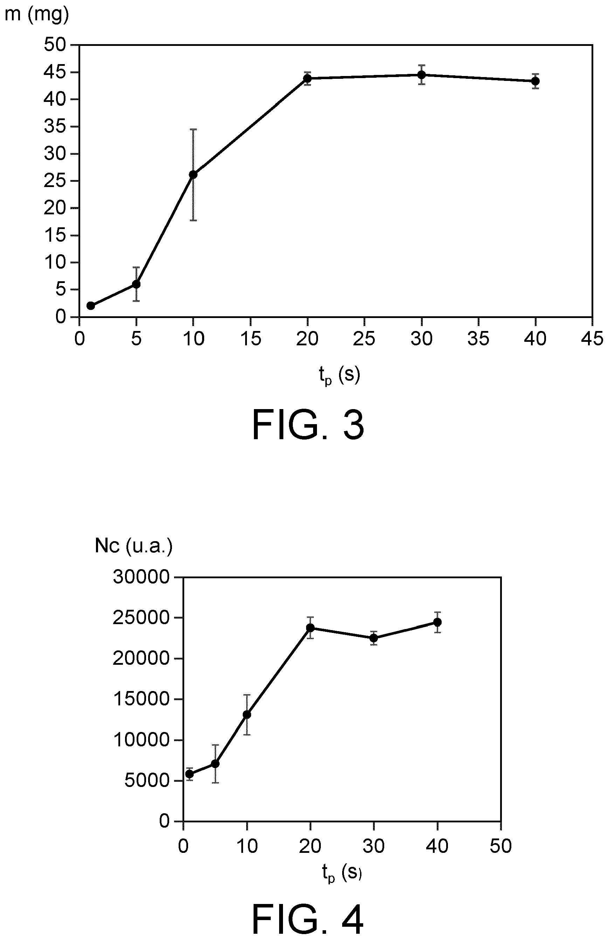

La figure 3 illustre l'évolution de la masse, notée m et exprimée en mg, de tissu adipeux prélevé au moyen d'un écouvillon en fonction de la durée, notée tₚ et exprimée en secondes, du prélèvement, c'est-à-dire en fonction du temps de contact entre l'écouvillon et le tissu adipeux.
La figure 4 illustre l'évolution de l'intensité maximale des signaux de luminescence obtenus en soumettant, à une réaction d'ECL par saut de potentiel, des extraits organiques en fonction de la durée du prélèvement ; sur cette figure, l'axe des ordonnées correspond au nombre de coups émis, noté Ne et exprimé en unités arbitraires (u.a.) tandis que l'axe des abscisses correspond à la durée de prélèvement, notée tₚ et exprimée en secondes.

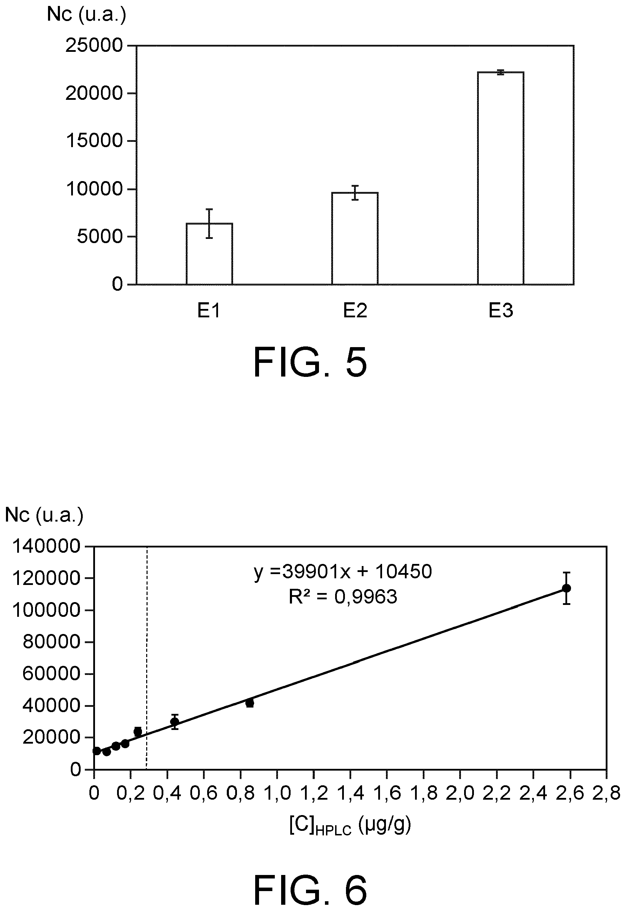

La figure 5 illustre, sous la forme d'un diagramme en bâtons, l'intensité maximale des signaux de luminescence obtenus en soumettant, à une réaction d'ECL par saut de potentiel, 3 extraits organiques, respectivement E1, E2 et E3, résultant d'une mise en solution d'échantillons de tissu adipeux par des modalités différentes; sur cette figure, l'axe des ordonnées correspond au nombre maximal de coups émis, noté Ne et exprimé en unités arbitraires (u.a.).
La figure 6 illustre les résultats d'un test visant à comparer le dosage par ECL du scatol dans des extraits organiques préparés conformément à l'invention à partir de 8 échantillons de tissus adipeux de porcs mâles entiers avec le dosage par HPLC du scatol dans le tissu adipeux de ces mêmes porcs ; sur cette figure, l'axe des ordonnées correspond au nombre de coups émis, noté Ne et exprimé en unités arbitraires (u.a.) tandis que l'axe des abscisses correspond à la concentration de scatol trouvée par HPLC, notée [C]_{HPLC} et exprimée en µg/g.

### EXPOSÉ DÉTAILLÉ DE MODES DE MISE EN OEUVRE PARTICULIERS

Les réactions d'ECL, dont les résultats sont rapportés ci-après, ont été réalisées avec des cellules électrochimiques telles que celle référencée 20 dans la référence [1], cette cellule électrochimique étant décrite au point II.1 de la référence [1] et illustrée sur la figure 2 de cette référence.

Pour les mesures de l'émission lumineuse totale, des mesures électrochimiques et d'ECL ont été effectuées à l'aide d'un potentiostat/galvanostat Autolab^{™} PGSTAT128N (Autolab) ou d'un module photodétecteur PDM03-9107-USB (ET-Enterprises).

Pour l'obtention des données spectrales, les mesures d'ECL ont été effectuées à l'aide d'un potentiostat portable PalmSens4^{™} (PalmSens) et d'un spectrofluorimètre Fluoromax^{™} 4P (Horiba Jobin Yvon) qui permet de suivre en temps réel l'évolution de la luminescence grâce à un logiciel intégré.

Au début de chaque session de mesures, les électrodes ont été soigneusement nettoyées par activation électrochimique en les immergeant dans une solution comprenant 0,1 mol/L de TBAHFP dans de l'acétonitrile et en appliquant des impulsions de 0,5 secondes de 2 mA et -2 mA pendant 200 cycles.

Pendant les réactions d'ECL, les cellules électrochimiques ont été placées dans le noir absolu pour limiter le bruit de fond du photodétecteur ou du spectrofluorimètre.

Comme précédemment indiqué, le type de réaction d'ECL qui est privilégié dans le cadre de l'invention comprend l'application du potentiel cathodique à l'électrode de travail d'une cellule électrochimique pour introduire la formation d'ions superoxydes, suivie de l'application d'un potentiel anodique à cette même électrode pour introduire l'oxydation de la base conjuguée du scatol si celui-ci est présent dans l'extrait organique.

Comme également précédemment indiqué, on préfère que ceci soit réalisé par un saut de potentiel, c'est-à-dire en appliquant à l'électrode de travail un potentiel négatif constant, pendant une durée suffisante pour saturer la surface de cette électrode en ions superoxydes, puis en lui appliquant un potentiel positif constant, également pendant une durée suffisante pour saturer la surface de l'électrode de travail en scatol oxydé.

C'est donc ce type de protocole qui a été retenu pour toutes les réactions d'ECL dont les résultats sont rapportés ci-après.

En l'espèce, ces réactions ont été réalisées en appliquant à l'électrode de travail un potentiel négatif constant de -1,8 V pendant 40 secondes puis un potentiel positif constant de +0,8 V pendant 5 secondes.

La mesure de la luminescence émise a été lancée dès le début de l'application du potentiel négatif à l'électrode de travail.

### I - Influence de la présence d'eau dans le milieu réactionnel sur le signal de luminescence mesuré :

Afin d'apprécier l'influence de la présence d'eau dans le milieu réactionnel sur le signal de luminescence émis, 3 solutions synthétiques, respectivement S1, S2 et S3, ne différant les unes des autres que par leur teneur en eau sont soumises à une réaction d'ECL.

Ces solutions sont préparées en dissolvant du scatol de synthèse dans de l'acétonitrile pour leur conférer une concentration en scatol de 100 nmol/L, et en ajoutant aux solutions résultantes du TBAHFP pour leur conférer une concentration en sel de fond de 0,1 mol/L.

Puis de l'eau est ajoutée aux seules solutions S2 et S3, à hauteur de 1 % volumique pour la solution S2 et de 2 % volumiques pour la solution S3. La solution S1 est donc exempte d'eau.

Les résultats sont illustrés sur la figure 1 qui illustre les signaux de luminescence obtenus pour les 3 solutions.

Cette figure montre que, lorsque de l'eau est présente dans le milieu réactionnel, l'intensité maximale du signal émis est affaiblie du fait de l'interaction de l'eau avec les ions superoxydes. Ainsi, la solution S3, comprenant 2 % volumiques d'eau, conduit à un signal de luminescence dont le pic est affaibli d'environ 70 % par rapport à celui obtenu pour la solution S1, exempte d'eau.

Une quatrième solution synthétique S4, comprenant aussi 100 nmol/L de scatol dans de l'acétonitrile et 0,1 mol/L de TBAHFP, mais à laquelle est ajouté 0,1 % volumique d'eau est préparée et également soumise à une réaction d'ECL.

Comme visible sur la figure 2 qui illustre l'intensité maximale des signaux de luminescence obtenus pour les solutions S1 à S4, celle obtenue pour la solution S4 est similaire à celle obtenue pour la solution S1.

Ceci signifie que, si l'on souhaite obtenir un signal de luminescence optimisé, il convient que l'extrait organique soumis à la réaction d'ECL ne comprenne pas plus de 0,1 % volumique d'eau.

### II - Préparation d'extraits organiques :

Les essais décrits ci-après sont réalisés à partir de tissus adipeux de porcs mâles entiers et en utilisant comme écouvillons de prélèvement, des cotons-tiges de laboratoire.

### II.1 - Influence de la durée de prélèvement :

Afin d'apprécier l'influence de la durée de prélèvement d'un échantillon de tissu adipeux au moyen d'un écouvillon de prélèvement sur la masse de tissu adipeux prélevée, une série de prélèvements est réalisée en frottant un tissu adipeux, issu d'une même carcasse de porc mâle entier, pendant 1, 5, 10, 20, 30 ou 40 secondes avec l'un des embouts des cotons-tiges.

Les cotons-tiges sont pesés avant et après les prélèvements, ce qui permet de déterminer, pour chaque durée de prélèvement, la masse de tissu adipeux prélevée.

Comme visible sur la figure 3, qui illustre l'évolution de la masse de tissu adipeux prélevée en fonction de la durée des prélèvements, il existe une corrélation entre cette masse et cette durée puisque la masse de tissu adipeux prélevée augmente avec la durée de prélèvement mais jusqu'à un certain seuil.

En effet, cette figure montre qu'à partir de 20 secondes de prélèvement, la masse de tissu adipeux prélevée reste constante, égale à environ 45 mg. Ceci signifie que l'embout du coton-tige est saturé en tissu adipeux et qu'il ne permettra pas de prélever plus de 45 mg de tissu adipeux.

Ainsi, étant donné qu'un tissu adipeux de porc mâle entier comprend naturellement au plus 20 % massiques d'eau, un extrait organique obtenu par mise en solution de 45 mg de tissu adipeux dans 10 mL d'acétonitrile comprendra 0,09 mg d'eau (sous réserve que l'intégralité du tissu adipeux soit mis en solution) et présentera donc un pourcentage volumique d'eau de 0,09, c'est-à-dire inférieur au seuil précédemment évoqué de 0,1 %.

Dans ces conditions, aucune opération visant à déshydrater l'extrait organique (chauffage, addition d'un sel hygroscopique ou d'un tamis moléculaire, etc.) ne sera nécessaire, ce qui représente un gain précieux de temps.

Par ailleurs, chacun des cotons-tiges utilisés ci-avant est introduit dans un tube à essai contenant 10 mL d'acétonitrile, 0,387 mg (i.e. 0,1 mol/L) de TBAHFP et 900 mg de sulfate de sodium, plongé dans ce milieu et mis à tourner contre la paroi du tube à essai pendant au moins 20 secondes. Les tubes à essai sont ensuite soumis à une agitation du type vortex pendant 10 minutes, puis laissés au repos pour décantation.

Les surnageants de décantation sont soumis à une réaction d'ECL.

L'évolution de l'intensité maximale des signaux de luminescence obtenus en fonction de la durée du prélèvement est illustrée sur la figure 4.

Cette figure montre qu'il existe une corrélation entre la durée du prélèvement et l'intensité maximale du signal de luminescence mesurée. En effet, comme ce qui a été montré précédemment par la figure 3, plus la durée du prélèvement est longue, plus le signal émis est intense, ceci étant dû à l'augmentation de la masse de l'échantillon de tissu adipeux prélevée au moyen des cotons-tiges.

Cette figure confirme aussi qu'à partir de 20 secondes de prélèvement, les cotons-tiges sont saturés en tissu adipeux, l'intensité du signal de luminescence étant constante et maximale.

### II.2 - Influence des modalités de mise en solution de l'échantillon de tissu adipeux :

Une fois l'échantillon de tissu adipeux prélevé au moyen d'un écouvillon de prélèvement, il doit être mis en solution.

Différentes modalités de mise en solution d'un échantillon de tissu adipeux prélevé au moyen d'un coton-tige sont testées.

Pour ce faire, 3 extraits organiques, respectivement E1, E2 et E3, sont préparés :
- soit en introduisant le coton-tige dans un tube à essai contenant 10 mL d'acétonitrile et 0,387 mg de TBAHFP et en le laissant simplement tremper dans ce milieu (extrait E1) ;
- soit en introduisant le coton-tige dans un tube à essai contenant 10 mL d'acétonitrile et 0,387 mg de TBAHFP, en le plongeant dans ce milieu et en le faisant tourner contre la paroi du tube à essai (extrait E2) ;
- soit encore, en introduisant le coton-tige dans un tube à essai contenant 10 mL d'acétonitrile, 0,387 mg de TBAHFP et 900 mg de sulfate de sodium (jouant ici le rôle d'agent abrasif), en le plongeant dans ce milieu et le faisant tourner contre la paroi du tube à essai (extrait E3).

Les extraits organiques ainsi obtenus sont soumis à une réaction d'ECL.

Les résultats sont illustrés sur la figure 4 qui représente l'intensité maximale des signaux de luminescence obtenus pour les extraits organiques E1 à E3.

Cette figure montre que les modalités de mise en solution des échantillons de tissu adipeux ont une influence sur le signal émis.

L'intensité maximale du signal de luminescence est obtenue pour l'extrait organique E3, ce qui confirme que l'ajout d'un agent abrasif dans le solvant organique aprotique permet une meilleure mise en solution de l'échantillon de tissu adipeux, préalablement prélevé au moyen d'un écouvillon.

### III - Analyse de tissus adipeux de porcs mâles :

On prépare, à partir de 8 échantillons de tissus adipeux de porcs mâles entiers présentant des concentrations différentes en scatol, préalablement déterminées par HPLC, des extraits organiques par le procédé de l'invention.

Pour ce faire, un échantillon de chaque tissu adipeux est prélevé par frottement d'un coton-tige pendant 20 secondes. Puis, chaque coton-tige est introduit dans un tube à essai contenant 10 mL d'acétonitrile, 0,387 mg (i.e. 0,1 mol/L) de TBAHFP et 900 mg de sulfate de sodium, plongé dans ce milieu et mis à tourner contre la paroi du tube à essai pendant au moins 20 secondes. Les tubes à essai sont ensuite soumis à une agitation du type vortex pendant 10 minutes, puis laissés au repos pour décantation.

Les extraits ainsi obtenus sont ensuite soumis à une réaction d'ECL par saut de potentiel.

La figure 6 illustre l'intensité du signal de luminescence mesurée par ECL en fonction de la concentration en scatol trouvée par HPLC.

Cette figure montre qu'il existe une corrélation quasi parfaite (R²=0,9963) entre les intensités des signaux de luminescence obtenues par le procédé de l'invention et les concentrations du scatol préalablement déterminées par HPLC à partir des mêmes tissus adipeux.

Elle montre également que le procédé de l'invention permet de détecter des concentrations de scatol bien inférieures au seuil de rejet de 0,2 µg de scatol/g de tissu adipeux.

En outre, les 8 échantillons de tissus adipeux étant analysés 5 fois chacun sur une période de 3 jours, l'écart-type est inférieur à 10% pour chaque mesure.

Il en résulte que le procédé de l'invention est sensible, reproductible, fiable et, donc, parfaitement adapté à une détection et un dosage du scatol sur une chaîne d'abattage de porcs mâles entiers.

### RÉFÉRENCES CITÉES

[1] WO-A-2021/009438
[2] T. Okajima et T. Ohsaka, Journal of Electroanalytical Chemistry 2002, 523, 34-39
[3] US 9,121,107 B2

## Revendications

1. Procédé pour détecter la présence de scatol dans un tissu adipeux d'un porc mâle, qui comprend au moins les étapes consistant à :
a) préparer un extrait organique à partir d'un échantillon du tissu adipeux ;
b) soumettre l'extrait organique préparé à l'étape a) à une réaction d'électrochimioluminescence ; et
c) mesurer l'intensité de la luminescence pendant l'étape b) et, si l'intensité de luminescence mesurée dépasse une valeur seuil prédéterminée, déduire la présence de scatol dans l'échantillon de tissu adipeux ;
et qui est **caractérisé en ce que** l'étape a) comprend les sous-étapes consistant à :
i) prélever l'échantillon de tissu adipeux à l'aide d'un écouvillon de prélèvement ;
ii) dissoudre l'échantillon de tissu adipeux dans un milieu comprenant un solvant organique aprotique ;
iii) ajouter un sel de fond anhydre au solvant organique aprotique, cet ajout pouvant être réalisé avant ou après la sous-étape ii) ;
moyennant quoi on obtient l'extrait organique.

2. Procédé selon la revendication 1, dans lequel la sous-étape i) comprend un frottement du tissu adipeux avec l'écouvillon pendant un temps suffisant pour saturer l'écouvillon en tissu adipeux.

3. Procédé selon la revendication 2, dans lequel le tissu adipeux est frotté avec l'écouvillon de façon continue pendant au moins 20 secondes.

4. Procédé selon l'une quelconque des revendications 1 à 3, dans lequel le solvant organique aprotique est l'acétonitrile, le diméthylsulfoxyde, le carbonate de propylène ou la γ-butyrolactone, de préférence l'acétonitrile.

5. Procédé selon l'une quelconque des revendications 1 à 4, dans lequel le sel de fond est un tétrafluoroborate, un hexafluorophosphate ou un perchlorate de tétraalkylammonium dont le groupe alkyle comprend de 1 à 6 atomes de carbone, de préférence l'hexafluorophosphate de tétrabutylammonium.

6. Procédé selon l'une quelconque des revendications 1 à 5, dans lequel l'extrait organique préparé à l'étape a) comprend au plus 0,1 % volumique d'eau.

7. Procédé selon l'une quelconque des revendications 1 à 6, dans lequel un agent abrasif est ajouté au solvant organique aprotique avant de procéder à la sous-étape ii).

8. Procédé selon la revendication 7, dans lequel l'agent abrasif est une poudre constituée d'un sel insoluble dans le solvant organique aprotique, une poudre de silice, une poudre d'un oxyde métallique, une poudre de pierre ponce ou une poudre de terre de diatomée.

9. Procédé selon la revendication 8, dans lequel l'agent abrasif est du sulfate de sodium anhydre, du sulfate de potassium anhydre ou du sulfate de magnésium anhydre.

10. Procédé selon l'une quelconque des revendications 7 à 9, dans lequel la sous-étape ii) comprend :
- l'introduction de l'écouvillon dans un récipient dans lequel se trouve le milieu comprenant le solvant organique aprotique, l'agent abrasif et, éventuellement, le sel de fond anhydre ;
- l'immersion de l'écouvillon dans le milieu ; et
- le frottement de l'écouvillon contre la paroi du récipient pendant au moins 20 secondes.

11. Procédé selon la revendication 10, dans lequel la sous-étape ii) comprend de plus la mise sous agitation du récipient.

12. Procédé selon la revendication 10 ou la revendication 11, dans lequel la sous-étape ii) comprend de plus l'élimination de l'agent abrasif du milieu.

13. Procédé selon l'une quelconque des revendications 1 à 12, dans lequel, la présence de scatol ayant été déduite à l'étape c), on réalise de plus une quantification du scatol présent dans l'extrait organique par comparaison de l'intensité maximale de luminescence mesurée au cours de l'étape c) avec une courbe d'étalonnage, la quantification étant réalisée au cours de l'étape c) ou postérieurement à l'étape c).

14. Procédé de tri de carcasses de porcs mâles entiers, **caractérisé en ce qu'**il comprend la mise en oeuvre d'un procédé selon l'une quelconque des revendications 1 à 13.
